# EUROPEAN PATENT SPECIFICATION

(11) **EP 3 693 357 B1**
(45) Date of publication and mention of the grant of the patent: **15.05.2024**
(21) Application number: 18865219.2
(22) Date of filing: 04.10.2018
(51) Int. Cl.: C07D 207/28

(54) **METHOD FOR PRODUCING OPTICALLY ACTIVE PYRROLIDONE CARBOXYLIC ACID OR ALKALI METAL SALT THEREOF**
VERFAHREN ZUR HERSTELLUNG VON OPTISCH AKTIVER PYRROLIDONCARBONSÄURE ODER EINEM ALKALIMETALLSALZ DAVON
PROCÉDÉ DE PRODUCTION D'ACIDE PYRROLIDONECARBOXYLIQUE OPTIQUEMENT ACTIF OU D'UN SEL DE MÉTAL ALCALIN CORRESPONDANT

(30) Priority: 05.10.2017 JP 2017195265
(43) Date of publication of application: 12.08.2020
(73) Proprietor: AJINOMOTO CO., INC., Chuo-ku Tokyo 104-8315 (JP)
(72) Inventor: SUGIMOTO, Takanori, Kawasaki-shi Kanagawa 210-8681 (JP)
(74) Representative: Mewburn Ellis LLP
(86) International application number: PCT/JP2018/037171
(87) International publication number: WO 2019/070018

(56) References cited:
- JP-A- H0 314 559
- JP-A- S4 725 169
- JP-A- S4 994 664
- JP-A- H01 132 559
- JP-A- H01 313 459
- JP-A- H02 145 565
- JP-A- H03 168 240
- JP-A- H10 337 195
- JP-A- S51 110 559
- JP-A- 2005 330 275
- JP-B1- S3 817 678
- JP-B1- S4 327 859
- US-A- 4 946 968
- US-A- 4 952 706
- US-A1- 2003 018 202
- KUMBERGER OTTO ET AL: "Metal Binding by Amino Acids: Preparation and Crystal Structures of Lithium, Sodium, and Potassium Hydrogen Bis-L-pyroglutamate", CHEMISCHE BERICHTE, vol. 125, no. 8, 1 August 1992 (1992-08-01), pages 1829-1834, XP055812076, DE ISSN: 0009-2940, DOI: 10.1002/cber.19921250808

## Description

### [Technical Field]

The present invention relates to a production method of optically active alkali metal salt of 2-pyrrolidone-5-carboxylic acid.

### [Background Art]

Pyrrolidonecarboxylic acid (PCA) is a useful compound not only as a cosmetic material but also as a starting material of pharmaceutical products and the like. Pyrrolidonecarboxylic acid is synthesized by heating glutamic acid to allow self-cyclization reaction. As a production method, a direct heating method without solvent, a high-pressure and high-heating method in the presence of solvent, a subcritical pressure and heating method, and an enzymatical cyclization method are known.

JP-A-1-132560 (patent document 1) describes the direct heating method without solvent. The direct heating method without solvent includes heating a monovalent alkaline earth metal salt of glutamic acid in a solid form to a temperature between 120°C and 250°C until complete elimination of reaction water of intramolecular condensation. The direct heating method without solvent is apparently industrially convenient with high production efficiency. In this production method, however, the molten material becomes syrupy during the reaction, and is hardly solidified on the apparatus wall surface after cooling. Therefore, this production method is not necessarily convenient in handling the reaction product. Moreover, it is known that, when heated, the obtained solid pyrrolidonecarboxylic acid produces pyroglutamic anhydride (impurity) as a by-product (see JP-B-41-19431 (patent document 2)), and this production method is not superior in obtaining pyrrolidonecarboxylic acid with high chemical purity.

JP-B-37-17959 (patent document 3) describes a method of obtaining pyrrolidonecarboxylic acid by dissolving L-glutamic acid by heating to 190 to 200°C and removing generated water, and a method of further heating pyrrolidonecarboxylic acid to give a racemate. JP-A-2003-34680 (patent document 4) describes a method of reacting glutamic acid with high temperature and high-pressure water. In these production methods, an autoclave and the like are used to stand the high temperature and high pressure. Therefore, these production methods are not suitable for versatilely obtaining pyrrolidonecarboxylic acid having high chemical purity and high optical purity, since conventional facility with no pressure resistance cannot be used for the production, and control of reaction is difficult since the reaction proceeds even during vacuum-cooling of the reaction vessel.

JP-A-10-66566 (patent document 5) discloses an enzymatical cyclization method. It describes a method of obtaining pyrrolidonecarboxylic acid from glutamic acid at high reaction rate under normal pressure in mild pH and mild temperature ranges in water by an enzyme reaction. However, as described in Example 5 of JP-A-10-66566, this method produces pyrrolidonecarboxylic acid only at a yield of about 0.4 mg, 300 nmol per 10 ml of the reaction mixture, and cannot be suitable for industrial production.

JP-B-5935689 (patent document 6) describes a method for producing pyrrolidonecarboxylic acid or a salt thereof with high chemical purity and high optical purity by heating cyclization of glutamic acid under normal pressure. By this method, however, the optical purity is 99.0% at maximum, and an aqueous solution of pyroglutamic acid having a high optical purity is necessary as the starting material. Thus, a more convenient production method has been desired.

On the other hand, it is known that zinc pyrrolidonecarboxylate having a high optical purity can be produced conveniently (patent document 7). However, a method capable of producing pyrrolidonecarboxylic acid conveniently in a high yield without lowering the optical purity but by removing zinc from this product is not known. In such cases, a method for removing zinc with an ion exchange resin is known. However, it is difficult to reduce same to some tens of ppm.

Patent document 8 discloses alkali metal salts of 2-pyrrolidone-5-carboxylic acid prepared by heating the corresponding alkali metal salt of glutamic acid to a temperature between its melting point and 270°C until the water liberated by the intramolecular condensation has been completely eliminated.

As described above, a method for producing pyrrolidonecarboxylic acid or a salt thereof having high optical purity and high chemical purity (HPLC purity) is not known. High optical purity and high chemical purity are important mainly for pharmaceutical products, pesticides and the like from the aspect of side effects and have been strongly desired.

### [Document List]

### [Patent documents]

patent document 1: JP-A-H1-132560
patent document 2: JP-B-41-19431
patent document 3: JP-B-37-17959
patent document 4: JP-A-2003-34680
patent document 5: JP-A-H10-66566
patent document 6: JP-B-5935689
patent document 7: JP-A-2005-330275
patent document 8: US 4,946,968 A

### [SUMMARY OF THE INVENTION]

### [Problems to be Solved by the Invention]

Thus, the present invention aims to provide a method for producing an alkali metal salt of pyrrolidonecarboxylic acid having an extremely high chemical purity and an extremely high optical purity conveniently in good yield.

### [Means of Solving the Problems]

The present inventors have conducted intensive studies in an attempt to solve the aforementioned problems and found that an alkali metal salt of 2-pyrrolidone-5-carboxylic acid having an extremely high chemical purity and an extremely high optical purity can be produced conveniently in a good yield by removing zinc extremely efficiently by adding an alkali metal hydroxide such as sodium hydroxide, potassium hydroxide or the like to a solution of optically active zinc 2-pyrrolidone-5-carboxylate 2 hydrate, and filtering off the precipitate (zinc hydroxide), which resulted in the completion of the present invention.

That is, the present invention relates to the following.
[1] A method for producing an optically active alkali metal salt of 2-pyrrolidone-5-carboxylic acid, comprising a step of adding an alkali metal hydroxide to a solution of optically active zinc 2-pyrrolidone-5-carboxylate 2 hydrate, and filtering off the precipitate.
[2] The production method of [1], wherein the solution of the optically active zinc 2-pyrrolidone-5-carboxylate 2 hydrate is an aqueous solution.
[3] The production method of [1] or [2], wherein the optically active alkali metal salt of 2-pyrrolidone-5-carboxylic acid has an HPLC purity of not less than 99.5% and an optical purity of not less than 99.5%.
[4] The production method of any one of [1] to [3], wherein the optically active alkali metal salt of 2-pyrrolidone-5-carboxylic acid does not contain pyroglutamylglutamic acid as impurity.
[5] The production method of any one of [1] to [4], wherein the optically active alkali metal salt of 2-pyrrolidone-5-carboxylic acid contains 0.1 - 20 ppm of zinc.
   Other preferred embodiments are set out in the dependent claims. [0012] Also disclosed herein are the following.
[6] An aqueous composition comprising 40 - 70 wt% of an optically active pyrrolidonecarboxylic acid or an alkali metal salt thereof, wherein the optically active pyrrolidonecarboxylic acid or an alkali metal salt thereof has an HPLC purity of not less than 99.5%.
[7] The aqueous composition of [6], wherein the optically active pyrrolidonecarboxylic acid or an alkali metal salt thereof has an optical purity of not less than 99.5%.
[8] The aqueous composition of [6] or [7], wherein the optically active pyrrolidonecarboxylic acid or an alkali metal salt thereof is an optically active potassium pyrrolidonecarboxylate.
[9] The aqueous composition of any one of [6] to [8], wherein the optically active pyrrolidonecarboxylic acid or an alkali metal salt thereof does not contain pyroglutamylglutamic acid as impurity.
[10] The aqueous composition of any one of [6] to [9], wherein the optically active pyrrolidonecarboxylic acid or an alkali metal salt thereof has a zinc content of 0.1 - 20 ppm.

### [Effect of the Invention]

According to the present invention, a method capable of producing an alkali metal salt of a 2-pyrrolidone-5-carboxylic acid having an extremely high chemical purity and an extremely high optical purity conveniently in a good yield can be provided.

### [Description of Embodiments]

The present invention is explained in detail in the following.

The production method of the optically active alkali metal salt of 2-pyrrolidone-5-carboxylic acid of the present invention includes a step of adding an alkali metal hydroxide to a solution of optically active zinc 2-pyrrolidone-5-carboxylate 2 hydrate and filtering off the precipitate.

Being "optically active" in the present specification means that the amount of one of optically active forms (L-form and D-form) is higher than the other, and denies a racemate containing equivalent amounts of L-form and D-form. In addition, the "optical purity" in the case of L-form, for example, refers to a percentage of L-form relative to the total of the L-form and D-form. In the present invention, the optically active form is preferably an L-form.

An optically active zinc 2-pyrrolidone-5-carboxylate 2 hydrate can be produced by the method described in JP-A-2005-330275.

The optical purity of the optically active zinc 2-pyrrolidone-5-carboxylate 2 hydrate is preferably 100% - 99.0%, more preferably 100% - 99.3%, further preferably 100% - 99.5%, particularly preferably 100% - 99.7%, from the aspect of obtaining an alkali metal salt of a 2-pyrrolidone-5-carboxylic acid having a high optical purity.

A solvent of the solution of the optically active zinc 2-pyrrolidone-5-carboxylate 2 hydrate is not particularly limited as long as it can dissolve optically active zinc 2-pyrrolidone-5-carboxylate 2 hydrate. Examples include water, alcohol such as methanol, ethanol, isopropanol and the like, a mixed solvent thereof and the like. From the aspect of production efficiency, water is preferable. That is, the solution of the optically active zinc 2-pyrrolidone-5-carboxylate 2 hydrate is preferably an aqueous solution.

The concentration of the solution of the optically active zinc 2-pyrrolidone-5-carboxylate 2 hydrate is not particularly limited. From the aspect of production efficiency, it is preferably 5 - 30 wt%, more preferably 10 - 30 wt%, further preferably 15 - 25 wt%.

Examples of the alkali metal hydroxide include lithium hydroxide, sodium hydroxide, potassium hydroxide and the like. From the aspect of easy availability, sodium hydroxide or potassium hydroxide is preferable. The amount of the alkali metal hydroxide to be used is generally 1.85 - 2.00 equivalents, preferably 1.90 - 2.00 equivalents, more preferably 1.90 - 1.95 equivalents, to the optically active zinc 2-pyrrolidone-5-carboxylate 2 hydrate.

Alkali metal hydroxide can be added, for example, as a solid or an aqueous solution, or an alcohol solution of methanol, ethanol or the like to a solution of optically active zinc 2-pyrrolidone-5-carboxylate 2 hydrate. From the aspect of easy addition, it is preferably added as an aqueous solution.

The temperature at which alkali metal hydroxide is added is generally 5 - 95°C, preferably 15 - 90°C, further preferably 50 - 85°C, and is reacted at the same temperature for generally 0.5 - 20 hr, preferably 1.0 - 3.0 hr.

Addition of alkali metal hydroxide causes progression of a salt exchange reaction, and the precipitate (zinc hydroxide) produced thereby is filtered off. The precipitate can be filtered off by a conventional method.

By the above-mentioned step, an alkali metal salt of the optically active 2-pyrrolidone-5-carboxylic acid is obtained. The obtained alkali metal salt of the optically active 2-pyrrolidone-5-carboxylic acid can be converted to a free form of the optically active 2-pyrrolidone-5-carboxylic acid according to a conventional method.

The optically active alkali metal salt of 2-pyrrolidone-5-carboxylic acid obtained by the method of the present invention has a chemical purity (HPLC purity) of preferably not less than 99.5%, more preferably not less than 99.7%, further preferably not less than 99.9%, and an optical purity of preferably not less than 99.5%, more preferably not less than 99.6%, further preferably not less than 99.7%. As used herein, the chemical purity (HPLC purity) and the optical purity were measured by the method described in the following Examples.

The optically active alkali metal salt of 2-pyrrolidone-5-carboxylic acid obtained by the method of the present invention shows a weight ratio of its L-form and D-form (L-form:D-form) of preferably 99.5:0.5 - 100:0, more preferably 99.6:0.4 - 100:0, further preferably 99.7:0.3 - 99.9:0.1.

In one embodiment of the present invention, the optically active alkali metal salt of 2-pyrrolidone-5-carboxylic acid obtained by the method of the present invention does not contain pyroglutamylglutamic acid as impurity. The pyroglutamylglutamic acid is a compound represented by the following structural formula.

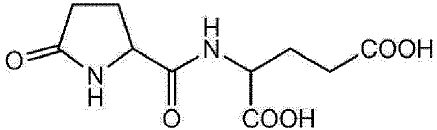

In another embodiment of the present invention, the optically active alkali metal salt of 2-pyrrolidone-5-carboxylic acid obtained by the method of the present invention contains zinc at preferably 0.1 - 20 ppm, more preferably 0.1 - 15 ppm, further preferably 0.1 - 12 ppm. As used herein, the content of zinc (residual zinc concentration) was measured by the method described in the following Examples.

The optically active alkali metal salt of 2-pyrrolidone-5-carboxylic acid obtained by the method of the present invention can be blended in cosmetics such as facial wash, soap, cleansing foam, skin lotion, skin milk, serum, beauty cream, shampoo, hair rinse, hair conditioner, enamel, foundation, chapstick, face powder, powder, facial mask, perfume, cologne, dentifrice and the like.

Conventional components generally used for cosmetics and skin external preparations can be used as an additive. Examples of the components generally used for cosmetics and skin external preparations include antioxidant, anti-inflammatory agent, UV absorber, whitening agent, hair-growth drug, cellular activator, moisturizing agent, metal chelating agent, oily starting material, surfactant, solvent, polymer substance, powder substance, dye, flavor, transdermal absorption promoter, antiseptic, discoloration preventives, buffers, medicaments for acne vulgaris, antidandruffs or antipruritics, antiperspirant deodorant, burn medicament, acaricides or pediculicides, keratin softeners, medicaments for xeroderma, antivirus agents, hormones, vitamins, amino acids, peptides, proteins, astringents, coolants or stimulators, components derived from animal or plant, antibiotics, antifungal agents and the like.

The present application also discloses an aqueous composition containing 40 - 70 wt% of an optically active pyrrolidonecarboxylic acid or an alkali metal salt thereof and having an HPLC purity of the optically active pyrrolidonecarboxylic acid or an alkali metal salt thereof of not less than 99.5%.

The aqueous composition is preferably an aqueous solution.

The optically active pyrrolidonecarboxylic acid or an alkali metal salt thereof contained in the aqueous composition can be preferably obtained, for example, by the production method of the optically active alkali metal salt of pyrrolidonecarboxylic acid of the present invention explained above.

The optically active pyrrolidonecarboxylic acid or an alkali metal salt thereof contained in the aqueous composition is preferably an optically active potassium pyrrolidonecarboxylate.

The content of the optically active pyrrolidonecarboxylic acid or an alkali metal salt thereof in the aqueous composition is 40 - 70 wt%, preferably 45 - 65 wt%, more preferably 45 - 60 wt%, based on the whole aqueous composition.

The aqueous composition may contain component other than the optically active pyrrolidonecarboxylic acid or an alkali metal salt thereof. Examples of such component include glutamic acid and a salt thereof.

### [Examples]

The present invention is explained in more detail in the following by referring to Examples, which are not to be construed as limitative.

### [Evaluation of chemical purity (indicated as HPLC purity) of optically active pyrrolidonecarboxylic acid or alkali metal salt thereof]

A sample obtained by synthesis was analyzed by HPLC, and the ratio of the peak area of pyrrolidonecarboxylic acid in the whole area was taken as the HPLC purity.
HPLC analysis conditions
column: Inertsil ODS-3 5 µm, 250×4.6 mm I.D.
eluent: 30 mM sodium dihydrogen phosphate (pH 3.0)
measurement temperature: 40°C
detection method: UV (210 nm)
software: LCsolution ver.1.25
peak position: peak appearing at RT about 7 min

In the evaluation, 99.8% or more is A, 99.0% or more is B, 95.0% or more is C, less than 90.0% is D.

### [Evaluation of optical purity (L-form purity) of optically active pyrrolidonecarboxylic acid or alkali metal salt thereof]

A sample obtained by synthesis was analyzed by HPLC using a chiral column, and the optical purity (L-form purity) was calculated according to the following calculation formula. HPLC analysis conditions
column: CHIRALPACK AS-H 5 µm, 250×4.6 mm I.D.
eluent: ethanol/trifluoroacetic acid = 100/0.1
measurement temperature: 30°C
detection method: UV (230 nm)
[L-form purity]=[L-form area]/([L-form area]+[D-form area])

In the evaluation, 99.5% or more is A, 99.0% or more is B, 98.0% or more is C, less than 98.0% is D.

### [Evaluation of residual zinc concentration]

A zinc content of a sample obtained by synthesis was calculated using PACKTEST (Kyoritsu Chemical-Check Lab., Corp.), converted to a concentration in the solid content and taken as the residual zinc concentration. The solid content was calculated by an infrared moisture meter.

In the evaluation, 20 ppm or less is A, 50 ppm or less is B, less than 100 ppm is C, and 100 ppm or more is D.

### <Example 1>

Optically active zinc pyrrolidonecarboxylate 2 hydrate (200 g; optical purity (L-form purity) 99.8%) was dissolved in ion exchange water (800 g), heated to 80°C, 48% aqueous potassium hydroxide solution (129.65 g) was gradually added, and the mixture was reacted at the same temperature for 1 hr. The precipitate was filtered off with Kiriyama funnel, and the filtrate was concentrated to give an aqueous solution of the optically active potassium pyrrolidonecarboxylate (341.1 g) (yield 940). The solid content was calculated to find 52%. HPLC purity was 100.0%, optical purity (L-form purity) was 99.7%, and residual zinc concentration was 6 ppm. The optically active potassium pyrrolidonecarboxylate prepared by this method did not contain pyroglutamylglutamic acid.

### <Comparative Example 1>

L-pyrrolidonecarboxylic acid (50.0 g; optical purity (L-form purity) 97.8%) was added to ion exchange water (7.0 g) and dissolved by heating. Sodium glutamate monohydrate (64.0 g) was added and dissolved therein, and the mixture was reacted at 120°C for 5 hr to give an aqueous solution of sodium L-pyrrolidonecarboxylate (yield 94%). HPLC purity was 92.5%, and optical purity (L-form purity) was 97.5%. Pyroglutamylglutamic acid was detected in the aqueous solution of sodium L-pyrrolidonecarboxylate prepared by this method.

### <Reference Example 1>

Optically active zinc pyrrolidonecarboxylate 2 hydrate (30 g; optical purity (L-form purity) 99.7%) was dissolved in ion exchange water (270 g), Dowex(R)G26 hydrogen form (100 g) was added and the mixture was stirred at room temperature for 1.0 hr. After stirring, the reaction mixture was filtered to give an aqueous solution of optically active pyrrolidonecarboxylic acid (303.0 g) (yield 920). The solid content was calculated to find 6.8%. HPLC purity was 100.0%, optical purity (L-form purity) was 99.8%, and residual zinc concentration was 368 ppm.

The evaluation of the HPLC purity, L-form purity and residual zinc concentration are summarized in the following Table.

**[Table 1]**

| | Example 1 | Comparative Example 1 | Reference Example 1 |
|---|---|---|---|
| HPLC purity | A 100.0% | C 92.5% | A 100.0% |
| L-form purity | A 99.7% | C 97.5% | A 99.8% |
| residual zinc concentration | A 6 ppm | - | D 368 ppm |
| comprehensive evaluation | A | D | - |

From Table 1, it has been clarified that an optically active pyrrolidonecarboxylic acid or an alkali metal salt thereof having a good HPLC purity and a good L-form purity can be prepared by adding alkali metal hydroxide to a solution of optically active zinc pyrrolidonecarboxylate 2 hydrate and filtering off the precipitate.

Such optically highly active pyrrolidonecarboxylic acid is useful for pharmaceutical products and pesticides required to show a high purity.

### [Industrial Applicability]

The present invention provides a method capable of producing an alkali metal salt of a 2-pyrrolidone-5-carboxylic acid having an extremely high chemical purity and an extremely high optical purity conveniently in a good yield. Such alkali metal salt of 2-pyrrolidone-5-carboxylic acid is considered to be useful as a product or a starting material in industries requiring high purity such as pharmaceutical products, pesticides and the like.

This application is based on patent application No. 2017-195265 filed in Japan.

## Claims

1. A method for producing an optically active alkali metal salt of 2-pyrrolidone-5-carboxylic acid, comprising a step of adding an alkali metal hydroxide to a solution of optically active zinc 2-pyrrolidone-5-carboxylate 2 hydrate, and filtering off the precipitate.

2. The production method according to claim 1, wherein the solution of the optically active zinc 2-pyrrolidone-5-carboxylate 2 hydrate is an aqueous solution.

3. The production method according to claim 1 or 2, wherein the optically active alkali metal salt of 2-pyrrolidone-5-carboxylic acid has an HPLC purity of not less than 99.5% and an optical purity of not less than 99.5%.

4. The production method according to any one of claims 1 to 3, wherein the optically active alkali metal salt of 2-pyrrolidone-5-carboxylic acid does not contain pyroglutamylglutamic acid as impurity.

5. The production method according to any one of claims 1 to 4, wherein the optically active alkali metal salt of 2-pyrrolidone-5-carboxylic acid contains 0.1 - 20 ppm of zinc.

6. The production method according to any one of claims 1 to 5, wherein the alkali metal hydroxide is sodium hydroxide or potassium hydroxide.

7. The production method according to any one of claims 1 to 6, wherein the concentration of the solution of the optically active zinc 2-pyrrolidone-5-carboxylate 2 hydroate is 5 - 30 wt%.

8. The production method according to any one of claims 1 to 7, wherein the optically active alkali metal salt of 2-pyrrolidone-5-carboxylic acid shows a weight ratio of its L-form:D-form of 99.5:0.5 - 100:0.

## Patentansprüche

1. Verfahren zur Herstellung eines optisch aktiven Alkalimetallsalzes von 2-Pyrrolidon-5-carbonsäure, umfassend einen Schritt des Zugebens eines Alkalimetallhydroxids zu einer Lösung von optisch aktivem Zink-2-pyrrolidon-5-carboxylat-2-hydrat und des Abfiltrierens des Niederschlags.

2. Herstellungsverfahren nach Anspruch 1, wobei die Lösung des optisch aktiven Zink-2-pyrrolidon-5-carboxylat-2-hydrats eine wässrige Lösung ist.

3. Herstellungsverfahren nach Anspruch 1 oder 2, wobei das optisch aktive Alkalimetallsalz von 2-Pyrrolidon-5-carbonsäure eine HPLC-Reinheit von mindestens 99,5 % und eine optische Reinheit von mindestens 99,5 % aufweist.

4. Herstellungsverfahren nach einem der Ansprüche 1 bis 3, wobei das optisch aktive Alkalimetallsalz von 2-Pyrrolidon-5-carbonsäure keine Pyroglutamylglutaminsäure als Verunreinigung enthält.

5. Herstellungsverfahren nach einem der Ansprüche 1 bis 4, wobei das optisch aktive Alkalimetallsalz von 2-Pyrrolidon-5-carbonsäure 0,1 - 20 ppm Zink enthält.

6. Herstellungsverfahren nach einem der Ansprüche 1 bis 5, wobei das Alkalimetallhydroxid Natriumhydroxid oder Kaliumhydroxid ist.

7. Herstellungsverfahren nach einem der Ansprüche 1 bis 6, wobei die Konzentration der Lösung des optisch aktiven Zink-2-pyrrolidon-5-carboxylat-2-hydrats 5 - 30 Gew.-% beträgt.

8. Herstellungsverfahren nach einem der Ansprüche 1 bis 7, wobei das optisch aktive Alkalimetallsalz der 2-Pyrrolidon-5-carbonsäure ein Gewichtsverhältnis seiner L-Form:D-Form von 99,5:0,5 - 100:0 aufweist.

## Revendications

1. Procédé pour produire un sel de métal alcalin et d'acide 2-pyrrolidone-5-carboxylique optiquement actif, comprenant une étape d'addition d'un hydroxyde de métal alcalin à une solution de 2-pyrrolidone-5-carboxylate de zinc dihydraté optiquement actif, et de retrait du précipité par filtration.

2. Procédé de production selon la revendication 1, dans lequel la solution de 2-pyrrolidone-5-carboxylate de zinc dihydraté optiquement actif est une solution aqueuse.

3. Procédé de production selon la revendication 1 ou 2, dans lequel le sel de métal alcalin et d'acide 2-pyrrolidone-5-carboxylique optiquement actif a une pureté par CLHP non inférieure à 99,5 % et une pureté optique non inférieure à 99,5 %.

4. Procédé de production selon l'une quelconque des revendications 1 à 3, dans lequel le sel de métal alcalin et d'acide 2-pyrrolidone-5-carboxylique optiquement actif ne contient pas d'acide pyroglutamylglutamique en tant qu'impureté.

5. Procédé de production selon l'une quelconque des revendications 1 à 4, dans lequel le sel de métal alcalin et d'acide 2-pyrrolidone-5-carboxylique optiquement actif contient 0,1 à 20 ppm de zinc.

6. Procédé de production selon l'une quelconque des revendications 1 à 5, dans lequel l'hydroxyde de métal alcalin est l'hydroxyde de sodium ou l'hydroxyde de potassium.

7. Procédé de production selon l'une quelconque des revendications 1 à 6, dans lequel la concentration de la solution de 2-pyrrolidone-5-carboxylate de zinc dihydraté optiquement actif est de 5 à 30 % en poids.

8. Procédé de production selon l'une quelconque des revendications 1 à 7, dans lequel le sel de métal alcalin et d'acide 2-pyrrolidone-5-carboxylique optiquement actif présente un rapport en poids de sa forme L à sa forme D de 99,5/0,5 à 100/0.
